# EUROPEAN PATENT APPLICATION

(11) **EP 4 717 307 A1**
(43) Date of publication of application: **01.04.2026**
(21) Application number: 25204584.4
(22) Date of filing: 25.09.2025
(51) Int. Cl.: A61N 5/10

(54) **METHOD AND APPARATUS TO FACILITATE OPTIMIZING A RADIATION TREATMENT PLAN**

(30) Priority: 26.09.2024 US 202463699496 P; 07.08.2025 US 202519293094
(71) Applicant: Siemens Healthineers International AG, 6312 Steinhausen (CH)
(72) Inventor: KIHNIA, Veli-Pekka, 02770 Espoo (FI); HALKO, Lauri, 00640 Helsinki (FI); KORHONEN, Laura, 02340 Espoo (FI); KEMPPAINEN, Matti, 04420 Järvenpää (FI); KURKI, Jani, 00670 Helsinki (FI)
(74) Representative: Mathisen & Macara LLP

(57) **Abstract**

A control circuit (101) (which may comprise a graphics processing unit) accesses (201) a plurality of radiation treatment planning objectives for a particular patient. The control circuit (101) then optimizes (202) a first candidate radiation treatment plan for the particular patient as a function of those objectives. The control circuit (101) can then detect (203) modification of at least one of those objectives to provide a corresponding one or more modified radiation treatment planning objective. The control circuit (101) can then optimize (206) a second candidate radiation treatment plan as a function of the at least one modified objective. The control circuit (101) can then present (207), via a shared user display, at least a first dose volume histogram (302) that corresponds to the first treatment plan and at least a second dose volume histogram (501) that corresponds to the second treatment plan. The first (302) and second (501) dose volume histograms can be visually distinguished from one another by other than color.

## Description

### CROSS-REFERENCE TO RELATED APPLICATIONS

This application claims priority from U.S. Provisional Application No. 63/699,496, filed September 26, 2024, and US Patent Application No. 19/293,094, filed 7 August 2025, which are hereby incorporated herein by reference in their entirety.

### TECHNICAL FIELD

These teachings relate generally to treating a patient's planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Clinical goals are the treatment goals being prescribed by, for example, the attending oncologist. Examples of clinical goals include, but are not limited to, goals regarding the dose distributions to be achieved with respect to a target volume, one or more organs-at-risk (OAR) in the vicinity of the target volume, or other specified or unspecified normal tissues. By their very nature, clinical goals are typically agnostic with respect to what physical radiation treatment platform serves to administer the radiation.

Optimization objectives, on the other hand, are typically based on clinical goals and provide a measure by which an optimization process can test or assure that a particular prescribed dose is being uniformly administered through the patient's target volume while avoiding undue dosing of other patient tissues (or, in other cases, that a series of dose histograms that specify acceptable dosing ranges for a variety of locations both in and external to the target volume are met).

Accordingly, optimization objectives will be understood to be objectives that are very much specifically designed to reflect and accommodate the technical details and specifications of a particular radiation treatment platform, specific details regarding the patient's presentation, and/or other physical details pertaining to a particular application setting. Such details are generally viewed as being outside the expertise and knowledge base of the person who prescribes the radiation treatment in the first place (i.e., for example, the aforementioned attending oncologist). As a result, the person prescribing the radiation treatment ordinarily does not also create the optimization objectives.

### SUMMARY OF INVENTION

In accordance with a first aspect of the invention, there is provided a method as defined by claim 1.

In accordance with a second aspect of the invention, there is provided an apparatus as defined by claim 14.

Optional features are defined by the dependent claims.

### BRIEF DESCRIPTION OF DRAWINGS

Various needs corresponding to the above are at least partially met through provision of the method and apparatus to facilitate optimizing a radiation treatment plan described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a screenshot as configured in accordance with various embodiments of these teachings;
FIG. 4 comprises a screenshot as configured in accordance with various embodiments of these teachings; and
FIG. 5 comprises a screenshot as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, pursuant to these various embodiments, a control circuit (which may comprise a graphics processing unit) accesses a plurality of radiation treatment planning objectives for a particular patient. The control circuit then optimizes a first candidate radiation treatment plan for the particular patient as a function of those objectives. The control circuit can then detect modification of at least one of those objectives to provide a corresponding one or more modified radiation treatment planning objective. The control circuit can then optimize a second candidate radiation treatment plan for the particular patient as a function of the at least one modified radiation treatment planning objective. The control circuit can then present, via a shared user display, at least a first dose volume histogram that corresponds to the first candidate radiation treatment plan and at least a second dose volume histogram that corresponds to the second candidate radiation treatment plan. By one approach, the first and second dose volume histograms can be visually distinguished from one another by other than color.

By one approach, the aforementioned first dose volume histogram and the second dose volume histogram are each presented using a similar (or even identical) color. By one approach, one of these histograms (such as the second dose volume histogram) is presented using a solid line while the remaining histogram (such as the first dose volume histogram) is presented using a broken line.

These teachings will accommodate using graphic icons that correspond to the aforementioned optimization objectives. In such a case, and by one approach, a graphic icon that corresponds to the modified radiation treatment planning objective can be presented in at least a partially duplicated form.

So configured, a user can more intuitively and easily quantify the impact of changing an optimization objective. These teachings will also accommodate allowing the user to quantify the impact of an optimization objective change to the optimization results without exiting the optimization workspace and without needing to create a copy of the plan.

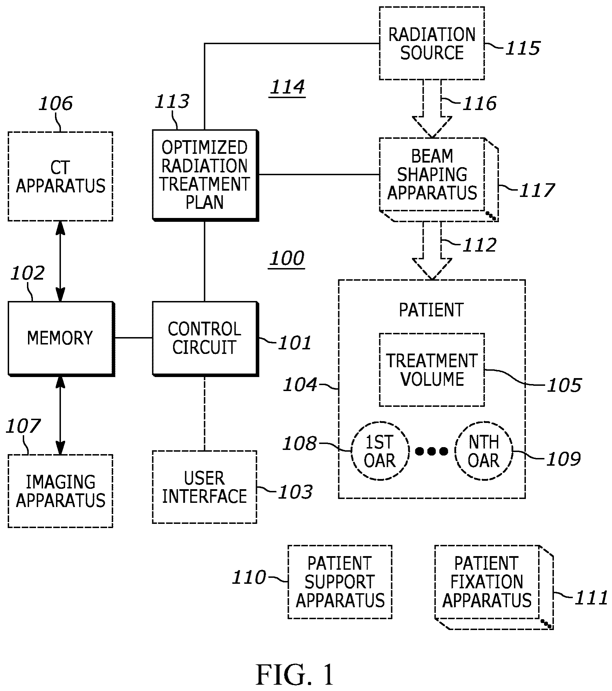

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

This control circuit 101 may comprise, for example, a central processing unit or a graphics processing unit. A graphics processing unit is a specialized electronic circuit designed to rapidly manipulate and alter memory to accelerate the creation of images in a frame buffer intended for output to a display device. Originally intended to offload the complex task of rendering computer graphics from a central processing unit, graphics processing units have evolved to become highly efficient at processing large blocks of data in parallel, which makes them particularly effective for a variety of computing needs.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient (including radiation treatment planning objectives) and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

In this illustrative example, the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

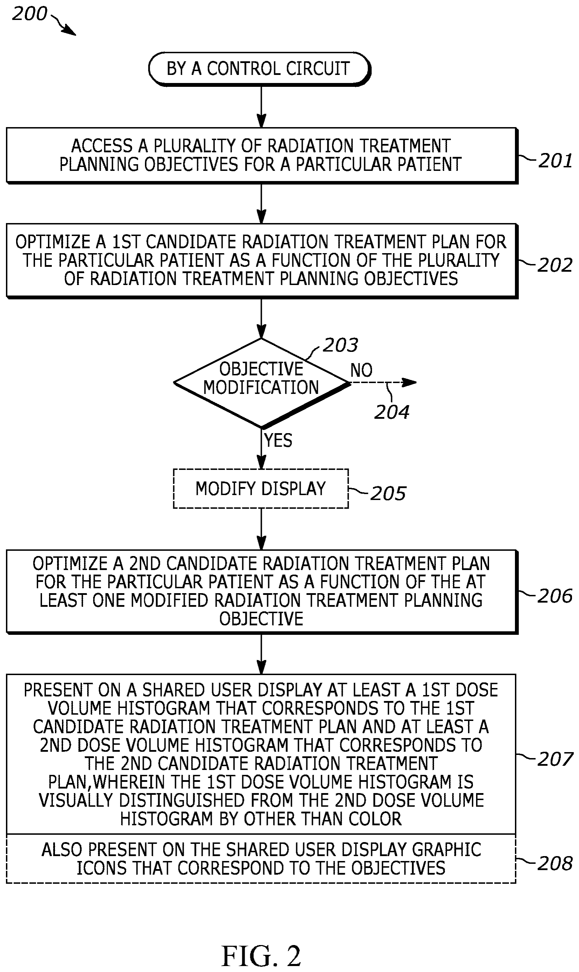

Referring now to FIG. 2, a process 200 that can be carried out, for example, in conjunction with the above-described application setting (and more particularly via the aforementioned control circuit 101 which, for the sake of an illustrative example and without intending any limitations in these regards, will be presumed to comprise a graphics processing unit in the following description) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for accessing a plurality of radiation treatment planning objectives for a particular patient. The control circuit 101 may access this information, by one approach, by accessing the aforementioned memory 102. By one approach, one or more of these radiation treatment planning objectives was generated by a human user. By another approach, in lieu of the foregoing or in combination therewith, one or more of these radiation treatment planning objectives was generated automatically. Various approaches for automatically generating radiation treatment planning objectives are known in the art. Since the present teachings are not overly sensitive to any particular selections in these regards, further elaboration regarding the automatic generation of radiation treatment planning objectives is not provided here for the sake of brevity.

At block 202, the control circuit 101 optimizes a first candidate radiation treatment plan for the particular patient as a function of the aforementioned plurality of radiation treatment planning objectives. Various approaches are known in the art to carry out such optimization. Many such approaches utilized so-called "cost constraints." In the context of an iterative optimization method, cost constraints refer to a predefined limitation that regulates the extent of one or more resources that can be allocated towards achieving the desired optimization objectives. Such constraints are factored into the optimization process to at least bias the solution towards adhering to boundaries set by such constraints. Accordingly, while the optimization process iteratively searches for an optimal solution by adjusting variables and assessing outcomes, the process simultaneously works to ensure that the total cost incurred in reaching that solution does not exceed the set cost constraint, thereby balancing twin goals of optimizing the treatment of the targeted patient volume while minimizing collateral harm to other tissue.

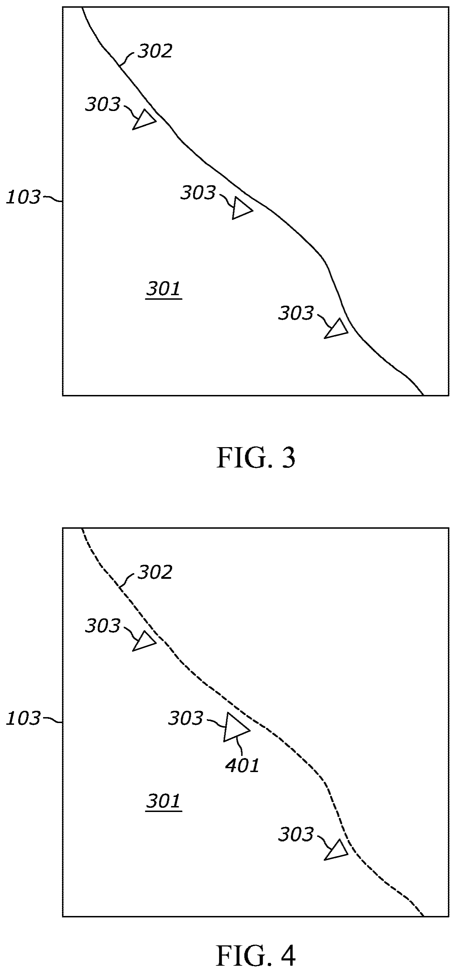

If desired, and as illustrated at FIG. 3, the control circuit 101 can present, via the aforementioned user interface 103, a display 301 that comprises one or more corresponding dose volume histogram lines 302. (FIG. 3 only depicts one such dose volume histogram line 302 For the sake of clarity. In a typical application setting, however, such a display 301 would present a dose volume histogram line for each of a plurality of patient volumes of interest, including a patient planning volume (such as, for example, a targeted tumor) and one or more organs-at-risk.) In this example, the display 301 also includes graphic icons 303 that correspond to particular optimization objectives. In this illustrative example, each such graphic icon 303 comprises an arrowhead.

At block 203, the control circuit 101 monitors for a modification to one of the radiation treatment planning objectives (including, in particular, a user modification effected, for example, via the aforementioned user interface 103). In the absence of detecting a trigger event, and as suggested at reference numeral 204, this process 200 can accommodate any of a variety of responses. Examples of responses can include temporal multitasking (pursuant to which the control circuit 101 conducts other tasks before returning to again monitor for a trigger event) as well as continually looping back to essentially continuously monitor for the trigger event. These teachings will also accommodate supporting this detection activity via a real-time interrupt capability.

Upon detecting a modification of at least one of the plurality of radiation treatment planning objectives to thereby provide at least one modified radiation treatment planning objective, by one optional approach and as presented at optional block 205, prior to completing re-optimization as a function of the modified optimization objective, the control circuit 101 can modify the display of the aforementioned dose volume histogram line 302 to visually signal that a change has been made to an optimization objective. (If desired, the user interface 103 may also present other signals of such a change. Examples include, but are not limited to, an audio signal, a textual message advising the user of the change, and so forth.)

FIG. 4 presents an illustrative example in these regards. In this example, the previously presented dose volume histogram line 302 is now presented using a different form factor (in this example, using a dashed line instead of a solid line). (In this particular example, that dose volume histogram line 302 is otherwise the same as when originally presented. In other words, the two lines would otherwise substantively match one another if one were laid atop the other and thereby convey identical dosing information.)

FIG. 4 also illustrates that the graphic icon 303 for the particular optimization objective that was just now modified can also be presented using a differentiated form factor as denoted by reference numeral 401. In this example, the differentiated form factor is an enlarged arrowhead.

In any event, at block 206, the control circuit 101 optimizes a second candidate radiation treatment plan for the particular patient as a function of the at least one modified radiation treatment planning objective to thereby generate a second candidate radiation treatment plan. By one approach, this re-optimization process can be identical to the preceding optimization process with the exception of the modified optimization objective.

At block 207, the control circuit 101 presents, on a shared user display (where the aforementioned user interface 103 simultaneously displays these described information items), at least a first dose volume histogram that corresponds to a particular patient volume per the first candidate radiation treatment plan and at least a second dose volume histogram that corresponds to the same particular patient volume, albeit per the second candidate radiation treatment plan, wherein the first dose volume histogram is visually distinguished from the second dose volume histogram by other than color.

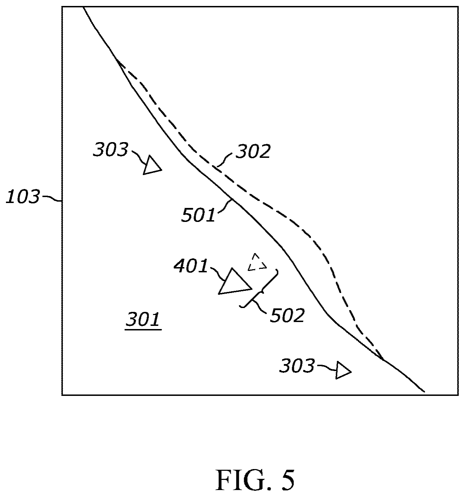

FIG. 5 presents an illustrative example. In this example, the user interface 103 presents a display 301 having both the above-described initial dose volume histogram line 302 presented as a dashed line and a second dose volume histogram line 501 presented as a solid line. The first dose volume histogram line 302 corresponds to the dose volume histogram for a particular patient volume per the first candidate radiation treatment plan. The second dose volume histogram line 501 corresponds to a dose volume histogram for the same patient volume but per the second candidate radiation treatment plan.

These teachings will accommodate, in lieu of the foregoing or in combination therewith, other approaches to visually differentiating the dose volume histogram lines 302 and 501 that correspond to the original optimization objective and the modified optimization objective, respectively. For example, one such line may be thicker than the other line. As another example, one line may use short dashes while the other line uses longer dashes. As yet another example, one such line may use a strobing effect that is different from the presentation of the other line. These examples are only intended to illustrate various possibilities and are not intended to suggest an exhaustive listing of all the ways that such lines may be visually distinguished from one another.

Many prior art approaches use color to differentiate dose volume histogram lines for different patient volumes. To help avoid confusion as regards dose volume histogram lines for these different patient volumes, the aforementioned first and second dose volume histogram lines 302 and 501 (which both correspond to the same patient volume) can both be presented using a similar or even an identical color.

As noted above, for the sake of clarity and simplicity, the foregoing examples present only a dose volume histogram line for one particular patient volume. These teachings, however, can be utilized in a similar manner with any number of dose volume histogram lines for any of a variety of patient volumes regarding which a radiation treatment plan is being optimized.

Also as noted above, these teachings will accommodate presenting graphic icons (such as the illustrated arrowheads 303) that are representative of (and potentially linked to) corresponding optimization objectives. In such a case, and as illustrated in FIG. 5, the graphic icon that corresponds to the modified optimization objective 401 can be presented in a modified form to reflect the modified state of the optimization objective itself. In this particular illustrative example, that modified form comprises presenting the graphic icon in at least a partially duplicate form 502 (in this case, a larger arrowhead and a smaller arrowhead positioned coaxially therewith). These teachings will accommodate other ways of distinguishing the graphic icon for the modified optimization objective from the original optimization objective, such as differentiated shading, differentiated color, differentiated transparency/opacity, animation, and so forth.

So configured, these teachings will accommodate using a fast graphics processing unit optimization algorithm as the optimization engine and then performing a quick optimization with initial values for the optimization objectives. When the user then makes a modification to one or more optimization objectives to seek to improve the dose distribution for a specific structure, these teachings can provide for automatically storing a snapshot of the dose volume histogram curves from the previous optimization for all structures and use those results as a reference for the following optimization.

After optimizing with the modified optimization objectives concludes, the control circuit 101 can display the reference dose volume histogram curves from the previous optimization in the same graphic presentation as the dose volume histogram curves from the current optimization. (In cases where there are multiple prior optimizations, these teachings will accommodate also presenting those earlier corresponding reference dose volume histogram curves and objectives. By one approach, the user can be provided with the opportunity to scroll between various prior optimizations to allow for separately displaying the results that correspond to each such prior optimization in comparison to the most recent optimization.) This approach makes evaluating how much the modification to the optimization objective changes the optimization results to thereby help the user understand and avoid, for example, unintended impacts to other structures. These teachings also make it considerably easier to quickly ascertain and understand which objective(s) were modified to reach the current results.

Further aspects of these teachings are provided by the subject matter of the following clauses (where it will be understood that any of these clauses can be combined with any one of more of the other clauses as appropriate). The clauses are subject matter of, but not currently claims of, the present specification.

Clause 1. A method to facilitate optimizing a radiation treatment plan for a particular patient using a particular radiation treatment platform, the method comprising: by a control circuit: accessing a plurality of radiation treatment planning objectives for the particular patient; optimizing a first candidate radiation treatment plan for the particular patient as a function of the plurality of radiation treatment planning objectives; detecting a modification of at least one of the plurality of radiation treatment planning objectives to provide at least one modified radiation treatment planning objective; optimizing a second candidate radiation treatment plan for the particular patient as a function of the at least one modified radiation treatment planning objective; presenting on a shared user display at least a first dose volume histogram that corresponds to the first candidate radiation treatment plan and at least a second dose volume histogram that corresponds to the second candidate radiation treatment plan, wherein the first dose volume histogram is visually distinguished from the second dose volume histogram by other than color.

Clause 2. The method of clause 1 wherein accessing the plurality of radiation treatment planning objectives for the particular patient comprises accessing a plurality of automatically generated radiation treatment planning objectives for the particular patient.

Clause 3. The method of either of clause 1 or 2 wherein detecting the modification of at least one of the plurality of radiation treatment planning objectives comprises detecting a user modification of at least one of the plurality of radiation treatment planning objectives.

Clause 4. The method of any of clause 1 through 3 wherein the control circuit comprises a graphics processing unit.

Clause 5. The method of any of clause 1 through 4 wherein the first dose volume histogram and the second dose volume histogram are each presented using a similar color.

Clause 6. The method of clause 5 wherein the first dose volume histogram and the second dose volume histogram are each presented using an identical color.

Clause 7. The method of any of clause 1 through 6 wherein one of the first dose volume histogram and the second dose volume histogram is presented using a solid line and another of the first dose volume histogram and the second dose volume histogram is presented using a broken line.

Clause 8. The method of clause 7 wherein the first dose volume histogram is presented using the solid line and the second dose volume histogram is presented using the broken line.

Clause 9. The method of any of clause 1 through 8 further comprising: also presenting on the shared user display graphic icons that correspond to the objectives.

Clause 10. The method of clause 9 wherein the graphic icon that corresponds to the modified radiation treatment planning objective is presented in at least a partially duplicate form.

Clause 11. An apparatus to facilitate optimizing a radiation treatment plan for a particular patient using a particular radiation treatment platform, the apparatus comprising: a control circuit configured to: access a plurality of radiation treatment planning objectives for the particular patient; optimize a first candidate radiation treatment plan for the particular patient as a function of the plurality of radiation treatment planning objectives; detect a modification of at least one of the plurality of radiation treatment planning objectives to provide at least one modified radiation treatment planning objective; optimize a second candidate radiation treatment plan for the particular patient as a function of the at least one modified radiation treatment planning objective; present on a shared user display that is operably coupled to the control circuit at least a first dose volume histogram that corresponds to the first candidate radiation treatment plan and at least a second dose volume histogram that corresponds to the second candidate radiation treatment plan, wherein the first dose volume histogram is visually distinguished from the second dose volume histogram by other than color.

Clause 12. The apparatus of clause 11 wherein the control circuit is configured to access the plurality of radiation treatment planning objectives for the particular patient by accessing a plurality of automatically generated radiation treatment planning objectives for the particular patient.

Clause 13. The apparatus of either of clause 11 or 12 wherein the control circuit is configured to detect the modification of at least one of the plurality of radiation treatment planning objectives by detecting a user modification of at least one of the plurality of radiation treatment planning objectives.

Clause 14. The apparatus of any of clause 11 through 13 wherein the control circuit comprises a graphics processing unit.

Clause 15. The apparatus of any of clause 11 through 14 wherein the first dose volume histogram and the second dose volume histogram are each presented using a similar color.

Clause 16. The apparatus of clause 15 wherein the first dose volume histogram and the second dose volume histogram are each presented using an identical color.

Clause 17. The apparatus of any of clause 11 through 16 wherein one of the first dose volume histogram and the second dose volume histogram is presented using a solid line and another of the first dose volume histogram and the second dose volume histogram is presented using a broken line.

Clause 18. The apparatus of clause 17 wherein the first dose volume histogram is presented using the solid line and the second dose volume histogram is presented using the broken line.

Clause 19. The apparatus of any of clause 11 through 18 wherein the control circuit is further configured to: also present on the shared user display graphic icons that correspond to the objectives.

Clause 20. The apparatus of clause 19 wherein the graphic icon that corresponds to the modified radiation treatment planning objective is presented in at least a partially duplicate form.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method to facilitate optimizing a radiation treatment plan for a particular patient using a particular radiation treatment platform, the method comprising:
by a control circuit:
accessing a plurality of radiation treatment planning objectives for the particular patient;
optimizing a first candidate radiation treatment plan for the particular patient as a function of the plurality of radiation treatment planning objectives;
detecting a modification of at least one of the plurality of radiation treatment planning objectives to provide at least one modified radiation treatment planning objective;
optimizing a second candidate radiation treatment plan for the particular patient as a function of the at least one modified radiation treatment planning objective;
presenting on a shared user display at least a first dose volume histogram that corresponds to the first candidate radiation treatment plan and at least a second dose volume histogram that corresponds to the second candidate radiation treatment plan, wherein the first dose volume histogram is visually distinguished from the second dose volume histogram by other than color.

2. The method of claim 1, further comprising automatically storing a snapshot of dose volume histograms from previous optimizations for one or more structures and displaying the dose volume histograms from previous optimizations in the same graphic presentation as a dose volume histogram from a current optimization.

3. The method of claim 1 or claim 2, wherein the detecting the modification comprises monitoring for a trigger event comprising a user modification effected via a user interface.

4. The method of claim 3, wherein in the absence of the trigger event, performing temporal multitasking, continually looping back to continuously monitor for the trigger event, and/or performing the detecting using a real-time interrupt capability.

5. The method of any preceding claim wherein accessing the plurality of radiation treatment planning objectives for the particular patient comprises accessing a plurality of automatically generated radiation treatment planning objectives for the particular patient.

6. The method of any preceding claim wherein detecting the modification of at least one of the plurality of radiation treatment planning objectives comprises detecting a user modification of at least one of the plurality of radiation treatment planning objectives.

7. The method of any preceding claim wherein the control circuit comprises a graphics processing unit.

8. The method of any preceding claim wherein the first dose volume histogram and the second dose volume histogram are each presented using a similar color.

9. The method of any preceding claim wherein the first dose volume histogram and the second dose volume histogram are each presented using an identical color.

10. The method of any preceding claim wherein one of the first dose volume histogram and the second dose volume histogram is presented using a solid line and another of the first dose volume histogram and the second dose volume histogram is presented using a broken line.

11. The method of any preceding claim wherein the first dose volume histogram is presented using a or the solid line and the second dose volume histogram is presented using a or the broken line.

12. The method of any preceding claim further comprising:
also presenting on the shared user display graphic icons that correspond to the objectives.

13. The method of claim 12 wherein the graphic icon that corresponds to the modified radiation treatment planning objective is presented in at least a partially duplicate form.

14. An apparatus to facilitate optimizing a radiation treatment plan for a particular patient using a particular radiation treatment platform, the apparatus comprising:
a control circuit configured to:
access a plurality of radiation treatment planning objectives for the particular patient;
optimize a first candidate radiation treatment plan for the particular patient as a function of the plurality of radiation treatment planning objectives;
detect a modification of at least one of the plurality of radiation treatment planning objectives to provide at least one modified radiation treatment planning objective;
optimize a second candidate radiation treatment plan for the particular patient as a function of the at least one modified radiation treatment planning objective;
present on a shared user display that is operably coupled to the control circuit at least a first dose volume histogram that corresponds to the first candidate radiation treatment plan and at least a second dose volume histogram that corresponds to the second candidate radiation treatment plan, wherein the first dose volume histogram is visually distinguished from the second dose volume histogram by other than color.

15. The apparatus of claim 14, further configured to perform the method of any of claims 2 to 13.
